(19) **Europäisches Patentamt**
**European Patent Office**
**Office européen des brevets**

(11) **EP 4 213 075 A1**

(12) **EUROPEAN PATENT APPLICATION**

(43) Date of publication:
**19.07.2023 Bulletin 2023/29**

(21) Application number: **22151155.3**

(22) Date of filing: **12.01.2022**

(51) International Patent Classification (IPC):
**G06N 3/08** *(2023.01)*        **G06N 3/04** *(2023.01)*
**G16H 70/60** *(2018.01)*        *G06N 5/02* *(2023.01)*

(52) Cooperative Patent Classification (CPC):
**G06N 3/08; G06N 3/045; G16H 70/60;** G06N 5/02

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer Aktiengesellschaft**
**51373 Leverkusen (DE)**

(72) Inventors:
• **Ziletti, Angelo**
**10553 Berlin (DE)**
• **Akbik, Alan**
**12049 Berlin (DE)**

(74) Representative: **BIP Patents**
**c/o Bayer Intellectual Property GmbH**
**Alfred-Nobel-Straße 50**
**40789 Monheim am Rhein (DE)**

(54) **MEDICAL CODING WITH BIOMEDICAL TRANSFORMER ENSEMBLES AND FEW-SHOT LEARNING**

(57) The invention relates to a computer-implemented solution for the attribution of a standardized hierarchical alphanumerical term and code (LLT) from an ontology of standardized terms and codes to a textual description of a medical event (RT), using one or more algorithm based on one or more pretrained transformer model(s) finetuned for mapping textual descriptions (RT) to the lowest level terms (LLT).

Fig. 2

**EP 4 213 075 A1**

**Description**

The invention relates to a computer-implemented solution for medical coding, that is for the translation of textual description of a medical event into a standardized alphanumerical term and code defined in a medical ontology.

Medical coding is the process of translating textual descriptions of medical events into standardized alphanumerical terms and codes. Examples of medical events are healthcare diagnosis, procedures, medical services, or equipment.

Medical coding is an essential pre-requisite for reliable data retrieval and reporting. It enables a variety of pharmaceutical company activities including but not limited to: operational coding support of pharmacovigilance and clinical studies; support of safety data retrieval, aggregation, and analysis for product safety labeling, including maintenance of existing labelling; provision, documentation and maintenance of product safety data sheets.

The core input of medical coding is the textual, that is natural language, description of the medical event, known as reported term (RT). The goal of medical coding is to associate a given RT to the most appropriate standardized term from a given medical ontology, e.g., MedDRA (MSSO, Retrieved Jun 24, 2021). An illustrative sample of the data is shown in Table 1.

In MedDRA, the most specific term in the hierarchy is the lowest level term (LLT). The preferred term (PT) is one level above the ontology hierarchy, and thus usually more general than the LLT. Each LLT is linked to only one PT. Each PT has at least one LLT (itself), but usually more than one LLTs are associated to a given PT. There are approximately 80,000 distinct LLT and 26,000 PT in MedDRA, which makes the correct assignment challenging. Regulatory authorities require the PT to be at correct at least 95% of the time at the end of the medical coding process, while there are no specific regulatory requirements for LLT accuracy.

The coding process is therefore cumbersome and for later treatment, in particular computer-implemented treatment, requires high quality with respect to consistency of the code assignment.

There was a need for a solution supporting a human coder in the coding process of medical information.

SUMMARY OF THE INVENTION

The problem was solved by use of state-of-the-art natural language processing, and in particular biomedical transformers, to generate high accuracy medical coding proposals that are then shown to human coders. These proposals support the medical coders, improving the efficiency of the coding process, and its quality with respect to consistency of the code assignment.

The solution of the invention relies on use of an algorithm, which is trained/able, given as input the RT, to generate the most appropriate LLT among the approximately 80,000 candidates from the chosen MedDRA ontology. This generated proposal will then be shown to the human coder, who will then perform the actual coding by either accepting the algorithm proposal or rejecting it. In case of rejection the user may choose to either introduce a more suitable LLT manually from the ontology or cause reiteration of the computer-implemented selection process.

The objects underlying the invention are achieved by the combination of features according to the independent Claims. Exemplary embodiments of the invention can be gathered from dependent claims thereto.

The invention will be more particularly elucidated below without distinguishing between the subjects of the invention. On the contrary, the following elucidations are intended to apply analogously to all the subjects of the invention, irrespective of in which context they occur.

BRIEF DESCRIPTION OF THE INVENTION

First object of the present invention is a computer-implemented method for attribution of a standardized hierarchical alphanumerical term and code from a ontology of standardized terms and codes organized in lowest level terms (LLT) to a textual description of a medical event (RT), comprising the steps of:

   a) Acquiring a textual description of a medical event (RT);

   b) Selecting one or more proposals for lowest level term (LLT) for the textual description of a medical event (RT) using one or more algorithm based on one or more pretrained transformer model(s) finetuned for mapping textual descriptions (RT) to a lowest level term (LLT);

   c) Optionally selecting one best matching proposal for lowest level term (LLT) by way of causing display of the selected one or more proposals for lowest level term (LLT) to a user for approval or rejection or automatically according to predefined criteria;

d) Saving the best matching proposal for lowest level term (LLT) to a record relating to the textual description of a medical event (RT).

The method of the invention relies on an algorithm trained for mapping RT to LLT. Such a mapping is already available for the coded and autocoded data, and thus these data sources can be readily used for training the algorithm.

A further object of the invention is a computer implemented method for the training of an algorithm for mapping a textual description of a medical event (RT) to lowest level terms (LLT) of an ontology, comprising the steps of:

    a. acquiring a training dataset of medical event records, the training set comprising records showing:

        - pairs of textual description of a medical event (RT) and corresponding lowest level terms (LLT) that have been defined by a human coder (also referred to as "coded data");

        - pairs of textual description of a medical event (RT) and corresponding lowest level terms (LLT) that have been coded by a rule-based autocoding system (also referred to as "autocoded data");

        - the ontology as medical event (RT) and lowest level terms (LLT); and /or

        - randomized pairs of textual descriptions of a medical event (RT) comprising one random character change and / or one word split and corresponding lowest level terms (LLT);

    b. feeding the training set of medical records to a pretrained transformer model for differential weighting of the significance of each part of the textual descriptions of a medical event (RT);
    c. initiating training of the model using a random seed to obtain a finetuned model for mapping a textual description (RT) with the level terms (LLT);
    d. saving the finetuned model for mapping the textual description RT to a best-matching lowest level term (LLT) for later use;
    e. reiterating step c) to d) for training of next model for mapping the textual description RT to a lowest level term LLT.

In an embodiment, the training dataset comprises "coded data", "autocoded data" and data obtained from one or more ontologies as further described below.

According to the present invention, the training dataset may comprise at least

- Coded data: pairs of RT and corresponding LLT, coded by a human coder.
- Autocoded data: pairs of RT and corresponding LLT, coded by a rule-based autocoding system. The rule-based autocoding system is designed to only autocode RTs which either are or contain a LLT verbatim. Thus, this system has a high precision, but a very low recall; the large majority of the samples are out of the autocoder scope, and thus passed to the humans for manual coding;
- Ontology is a list of standardized LLT, such as the one issued from the MedDRA (MSSO, Retrieved Jun 24, 2021), ICD9, ICD10, WHO DD, etc..., wherein each LLT can be used also as RT in the training dataset;

In a further embodiment, the training dataset further comprises "company synonyms".

- Company synonyms: pairs of medical text descriptions and corresponding LLT. These synonyms are created and maintained by a company medical coding department. These synonyms can define concepts that are more specific than their corresponding LLT in MedDRA. For example, focal nodular hyperplasia left liver lobe and focal nodular hyperplasia of right hepatic lobe are synonyms that refer to the LLT focal nodular hyperplasia.

For better training, it is preferred that the training dataset is as big as possible.

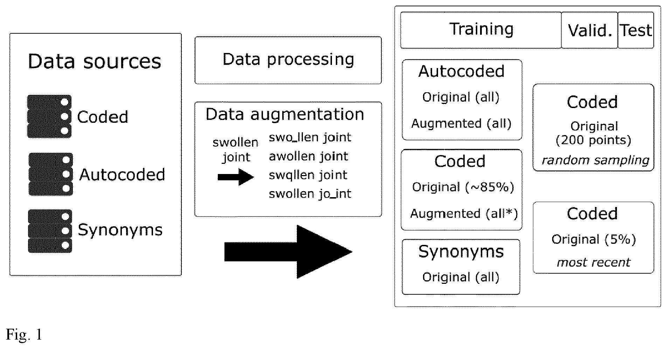

The training dataset can be augmented by using randomized pairs of textual descriptions of a medical event (RT) corresponding to a lowest level term (LLT). For example, for each LLT in the ontology, further three RTs can be generated for augmentation of the training dataset: the LLT verbatim, plus two simulated misspelled entries (one word split and one character change), as depicted in Fig. 1; all four RTs being paired with the original LLT as label. This procedure of adding all possible MedDRA LLT via simulated samples enables the algorithm to make predictions encompassing all possible LLTs, including the ones never encountered in the real (autocoded + coded) data.

The training dataset described above presents a large number of rare classes: more than 60% of the LLT appear less than five times in the data. To mitigate this problem, for each sample belonging to rare LLTs, we perform data augmentation by generating two simulated samples (one word split and one random character change, see Fig.

1). LLTs are defined as rare if they have less than ten samples.

Also, each company synonym can be used as RT, and the pair RT/LLT can be added to the training dataset.

In an embodiment, selecting the best-matching lowest level term (LLT) for the textual description of a medical event (RT) is achieved using a classification model, based on biomedical pretrained transformers.

The medical coding task can be framed as a multiclass classification task, where each possible LLT name from the MedDRA ontology is treated as a distinct class (~ 80,000 classes). As shown in Table 1, RTs usually comprise only a handful of words. This does not allow to learn the word semantic based on word co-occurrences, since words rarely occur together. Contextual word representations (M. E. Peters, et al. 2018. Deep contextualized word representations. In NAACL-HLT, pages 2227-2237. Association for Computational Linguistics; J. Devlin, et al. 2018. Bert: Pre-training of deep bidirectional transformers for language understanding. arXiv preprint arXiv: 1810.04805) from pretrained transformer models (A.Vaswani et al., 2017; Attention is all you need, Advances in Neural Information Processing Systems 30, pages 5998-6008. Curran Associates, Inc) learn word semantic directly from the large corpus on which they are trained on. They also have adequate performance in natural language processing tasks, including the biomedical domain (Y. Gu et al., 2020, Domain-specific language model pretraining for biomedical natural language processing. CoRR, abs/2007.15779).

A transformer model is a deep learning model that is differentially weighting the significance of each part of the input data, identifying the context that confers meaning to each word in the sentence. Transformer models have been pretrained with large language datasets. For example, BERT models (J. Devlin et al., 2018) have been applied to large-scale multi-label text classification for biomedical data, showing results comparable to more complex and bespoke approaches. They even exhibit competitive performance in the few-shot regime (I. Chalkidis et al., 2020, An empirical study on largescale multi-label text classification including few and zero-shot labels. In Proceedings of the 2020 Conference on Empirical Methods in Natural Language Processing (EMNLP), pages 7503-7515, Online. Association for Computational Linguistics).

In an embodiment, one or more pretrained transformer models can be used.

Specifically, best performing pretrained language/transformer models for biomedical tasks recommended by the BLURB leaderboard (Y. Gu et al., 2020) can be used.

- bioBERT (J. Lee et al., 2019, BioBERT: a pre-trained biomedical language representation model for biomedical text mining. Bioinformatics, 36(4): 1234-1240 ) is a BERT model trained on a biomedical corpus via a mixed domain pre-training strategy. The starting point is a standard BERT model pretrained on general corpus such as Wikipedia and BookCorpus. From that, the pretraining process is continued using biomedical text, namely PubMed abstracts (PubMed) and PubMedCentral (PMC) full text articles. BioBERT v.1.1 was used.

- PubMedBERT (Y. Gu et al., 2020) is a BERT model which - in contrast with bioBERT - is pretrained exclusively on biomedical text; specifically, it does not use the BERT weights as initialization, and it builds the vocabulary from scratch based on the biomedical text. The training corpus is also PubMed and PMC. A larger batch size w.r.t. bioBERT (8,192 vs 192) was used in the pretraining process.

- sciBERT (I. Beltagy et al., 2019, . SciBERT: A pretrained language model for scientific text.In Proceedings of the 2019 Conference on Empirical Methods in Natural Language Processing and the 9th International Joint Conference on Natural Language Processing (EMNLP-IJCNLP), pages 3615- 3620, Hong Kong, China. Association for Computational Linguistics) is a BERT model which is also pretrained on a corpus comprising both computer science (18%) and biomedical papers (82%) from Semantic Scholar (W. Ammar et al., 2018, Construction of the literature graph in semantic scholar. In Proceedings of the 2018 Conference of the North American Chapter of the Association for Computational Linguistics: Human Language Technologies, Volume 3 (Industry Papers), pages 84-91, New Orleans - Louisiana. Association for Computational Linguistics).

In an embodiment, one pretrained transformer is used.

In another embodiment, several pretrained transformer can be used. Most preferred are pretrained transformers established in the field of natural language processing, such as bioBERT, PubMedBERT and sciBERT.

To achieve a classification prediction from a language model, one needs to train (finetune) a further model to predict - given a RT as input - the corresponding LLT from the chosen ontology.

However, it was found that training a single model is not advisable for a production setting due to an underspecification problem. Underspecification is ubiquitous in deep learning applications, and transformers models are no exception (A. D'Amour et al., 2020, Underspecification presents challenges for credibility in modern machine learning. CoRR, abs/2011.03395 ). Models that perform equally well on their training domain can produce widely different results in their deployment domain, especially under dataset shift. This can result in instabilities and unexpected model behavior when models are deployed in a real-world setting.

In an embodiment, the one or more models for mapping textual descriptions (RT) to a best-matching lowest level term (LLT) are one or more trained deep ensembles (also called deep ensemble models) or one or more trained xTARS model algorithms.

In a further embodiment, one or more deep ensembles can be used for selection of a subset of best LLT predictions, and one or more xTARS models can be used to for mapping textual descriptions (RT) to a best-matching lowest level term (LLT) out of the subset.

In an embodiment, deep ensembles as described by Lakshminarayanan et al. can be used to mitigate the underspecification problem (B. Lakshminarayanan et al, 2017, Simple and scalable predictive uncertainty estimation using deep ensembles. In Proceedings of the 31st International Conference on Neural Information Processing Systems, NIPS 17, pages 6405-6416, Red Hook, NY, USA. Curran Associates Inc). The main idea is to train different models which only differ by a random perturbation (usually the random seed) and then average across these models to increase prediction stability, and possibly performance. Deep ensembles have also shown to produce reliable uncertainty estimates (B. Lakshminarayanan et al., 2017; S. Fort et al., 2020, Deep ensembles: A loss landscape perspective, arXiv: 1912.02757v2), on par with Bayesian deep learning approaches such as Monte Carlo dropout (Y. Gal et al, 2016, Dropout as a bayesian approximation: Representing model uncertainty in deep learning. In Proceedings of the 33rd International Conference on International Conference on Machine Learning - Volume 48, ICML'16, pages 1050-1059. JMLR.org ).

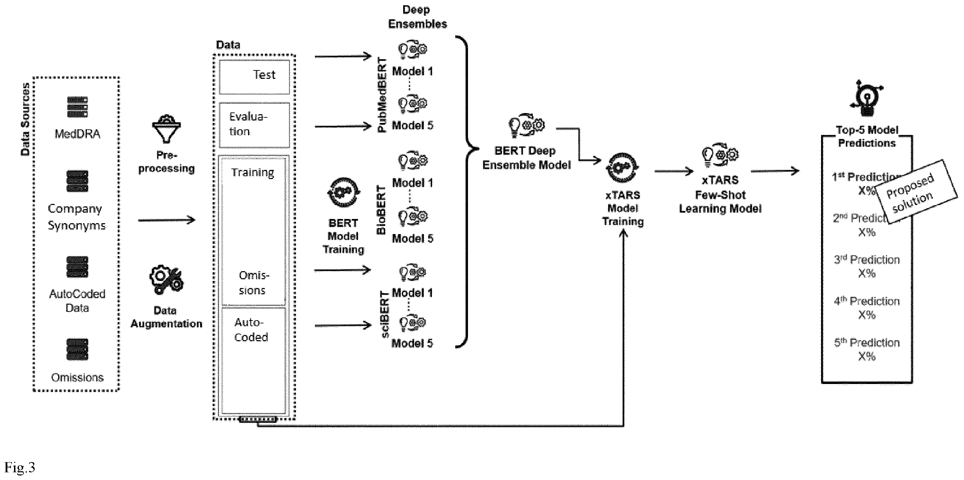

Preferred embodiment is depicted in Fig. 3.

In an embodiment, for each pretrained transformer model, one or more different models for mapping textual descriptions (RT) to a best-matching lowest level term (LLT) can be trained, which differ in the random seed initialization. In an embodiment, multiple models can be trained which differ only in the random seed initialization.

For this purpose, the method for training of an algorithm for mapping a textual description of a medical event (RT) to lowest level terms (LLT) described above can be reiterated for one or more for one or more random seeds, resulting for each random seed in multiple finetuned models for matching of a textual description (RT) with lowest level terms (LLT).

In an embodiment, for each pretrained transformer model, more than one random seed is used for training.

In an embodiment, for each random seed initialization, training is reiterated resulting in multiple finetuned models for matching of a textual description (RT) with lowest level terms (LLT) for each random seed.

In an example, training of each model can be performed on the training dataset for multiple epochs with Adam optimizer, which is a stochastic gradient descent method that is based on adaptive estimation of first-order and second-order moments. Best number of epochs were found to be from 5 to 40, best 20.

In an embodiment, each finetuned model for mapping a textual descriptions (RT) to the lowest level terms (LLT) is providing a probability vector of classification comprising a probability value for each lowest level term (LLT) of the ontology at stake.

In an embodiment, each finetuned model for mapping a textual description RT to lowest level terms LLT is validated, by way of:

- Acquiring a validation set of medical event records, the validation set comprising records wherein textual description of a medical event (RT) and corresponding lowest level terms (LLT) that have been defined by a human coder are selected in a randomly manner;
- Predicting for each record one best-matching lowest level term (LLT) for the textual description of a medical event (RT) using the fine-tuned model for mapping textual descriptions (RT) to lowest level terms (LLT), wherein said finetuned model is providing a probability vector of classification comprising a probability value for each lowest level term (LLT) of the ontology and the lowest level term (LLT) with highest probability value in the probability vector is selected,;
- For each record of the validation set, comparing the predicted LLT with acquired LLT of the validation set;
- Calculating the accuracy for the fine-tuned model at stake based on said comparison;
- For each random seed, selecting the fine-tuned models with highest accuracy value; and
- Saving the selected fine-tuned models for later use.

Wherein the term accuracy refers to the ratio of number of correct predictions to the total number of predictions.

In an embodiment, the finetuned model(s) with highest accuracy is selected for each random seed and saved for later use.

In an embodiment, the selected finetuned models for mapping RT and LLTs selected in the validation step described above are combined, so the most stable and best possible prediction algorithm is achieved.

In an embodiment, the selected finetuned models related to one transformer model can be combined in one ensemble (model).

In an embodiment, multiple pretrained transformer models are used and multiple models/ensembles for mapping a textual description (RT) with the level terms (LLT) are generated for each pretrained transformer model.

When BERT pretrained transformers are used, generated ensembles are generically referred to as BERT-ensemble(s).

In another embodiment, a method for a few-shot model can be used for mapping a textual description (RT) to a lowest level term (LLT).

Traditional machine learning algorithms do not have access to the natural language definition of the class (e.g., lung nodule) but rather to a discrete representation known as encoding. An established method of converting labels to prepare it for an algorithm is the one-hot encoding method, wherein each categorical value is converted into a new categorical column and a binary value of 1 or 0 is assigned to those columns; each integer value is represented as a binary vector.

In an embodiment, the one-hot encoding method is applied to the classes (LLT), thus obtaining a semantically-unaware class representation of the training set. Classes (LLT) are represented by a one-hot encoding vector with length equal to the number of possible classes in the ontology. In other words, if a sample belongs to the i-th class, then the i-th component of the one-hot encoding vector is set to one, while all other components are set to zero. This representation allows to keep track of classes during learning but does not preserve any semantic information (i.e., it is semantically unaware) present in the natural language definition. As a result, the model can learn the class meaning only indirectly, via the samples associated to a given class during learning.

Experience shows that a reliable model for mapping a RT with an LLT can be trained using a semantically-unaware class representation (e.g., one-hot encoding) of the training set when the number of samples per class is relatively large (say, larger than 20). However, when the samples per class are fewer, the lack of semantic class information is a clear drawback. In medical coding, the number of possible classes is very large, and it is essentially inevitable that a large number of classes only have few (e.g., less than 10) samples. These are the so-called rare classes.

To tackle the issue of rare classes, Halder et al. (K. Halder, 2020, Task-aware representation of sentences for generic text classification. In Proceedings of the 28th International Conference on Computational Linguistics, pages 3202-3213, Barcelona, Spain (Online). International Committee on Computational Linguistics ) proposed a specialized transformer model (Task-Aware Representation of Sentences, TARS) which retains the semantic class information, and thus it is able to learn effectively from few samples (few-shot learning). Inspired by natural language inference, TARS main idea is to concatenate the text to be classified (the reported term in our case) with class labels, and then predict True if the label is the correct one, and False otherwise. In the training process, for each sample, the model is shown the correct label and instructed to predict True, and a set of wrong labels (called negative samples) and instructed to predict False. These negative samples are drawn automatically from the available classes; the probability of choosing a given class is proportional to the cosine similarity between the correct class and a given class (Halder et al., 2020). In this way, negative classes that are more like the correct one (hence more difficult to distinguish) are drawn more often as negative samples. At prediction time, for each input text (e.g., reported term) the model will append each candidate class, and return the probability that the class is the correct one. The class with the highest probability is returned as predicted class. This procedure requires K predictions, where K is the number of classes. If K is large (e.g., K ~ 80,000 for medical coding with MedDRA), calculating predictions can be computationally prohibitive. The TARS algorithm proposed by Halder et al. was found to be not directly suitable to tackle large-scale classification tasks, as is the case of medical coding.

In an embodiment, one of the following improvements to the TARS algorithm - also referred to as negative sampling, prediction candidates, and adaptive number of negative samples - or combinations thereof can be used for large scale classification. Such improved algorithms are further described below and generically referred to as xTARS.

- Prediction candidates: at prediction /attribution time, instead of considering all candidate classes K (K ~ 80,000), only a subset of best lowest level term (LLT) predictions for example obtained using a trained deep ensemble model are considered in the xTARS model. Such a subset is also referred to as top-M candidates from the deep ensemble model, where M is typically between 5 and 20. This was found to lead to a three-order of magnitude reduction in computational cost, while resulting in only a small decrease in accuracy. In other words, this embodiment takes into consideration that the xTARS model cannot predict correctly if the correct class is not in the top-M candidate classes. M can be chosen so that a target user-specific cumulative accuracy (e.g., 95%) is reached by the deep ensemble model. Use of an xTARS model was found to improve performance of the method, even if the top-k accuracy of the deep ensemble model is already generally high.

- Negative sampling: the cosine similarity used in TARS returns a very broad similarity distribution, which means that also classes that are semantically very different from the correct one may have a relatively high similarity value. This similarity value can be used as probability of drawing the respective class as negative sample. In the case of large-scale classification, the model will often see negative classes that are obviously not correct, and thus it will have difficulties in learning to discriminate between the correct class, and classes similar to it, yet incorrect.

To solve this problem, the negative sampling procedure can be modified as follows:

- First, calculating the cosine similarity between the correct class and all available classes, like in the original TARS algorithm;

- Extracting only the top-L candidate classes, where L is typically between 10 and 50, and setting all other similarities to zero;

- Rescaling each similarity pi of the top-L classes via temperature-scaled softmax according to formula (2):

$$\bar{p}_i = \frac{e^{\frac{p_i}{T}}}{\sum_{j=1}^{l} e^{\frac{p_j}{T}}} \qquad (2)$$

High T will result in more peaked distribution (higher probability to more similar classes), while lower T will result in a broader distribution. T is a hyperparameter of x TARS; typical values of T are 0.01, 0.1 and 1

- Drawing classes with rescaled probability $\tilde{p}_i$ and using them as negative classes in the training procedure. In an embodiment, negative classes can be combined with the top-k predictions from the deep ensemble model (excluding the correct class) mentioned above for training of the algorithm.

This combination was found to improve the quality of the negative classes, ensuring that the model sees also classes that are very similar to the correct class, yet incorrect.

- Adaptive number of negative samples: in this embodiment, at training time, for each sample, negative samples are introduced, together with the correct label, into the xTARS model. In TARS, the model is shown the same number of negative samples for all samples. Here, instead, more (less) negative samples are shown to the model when the uncertainty of the deep ensemble model is higher (lower) to facilitate the learning of difficult samples. Specifically, a minimum (nnegmin) and a maximum number of negative samples (nnegmax) can be defined. Samples with uncertainty belonging to the first uncertainty quintile (low uncertainty) are shown nnegmin negative samples; samples within the last quintile (high uncertainty) are shown (nnegmax) negative samples. For samples with intermediate uncertainty, a linear interpolation between nnegmin and nnegmax can be performed depending on the uncertainty to obtain the number of negative samples to be shown.

The xTARS model can be trained a similar fashion to the deep ensemble model with the exception that only coded and autocoded data are used as training data (without data augmentation). This is to reduce computational cost.
In other words, the training of a xTARS model can comprise the steps of:

a. acquiring a training dataset of medical event records, the training set comprising records showing:

- pairs of textual description of a medical event (RT) and corresponding lowest level terms (LLT) have been defined by a human coder;

- pairs of textual description of a medical event (RT) and corresponding lowest level terms (LLT) have been coded by a rule-based autocoding system;

- optionally, negative samples;

b. feeding the training set of medical records to a pretrained transformer model for differential weighting of the significance of each part of the textual descriptions of a medical event (RT);
c. feeding the result of step b), finetuning of the pretrained transformer model to map the textual description (RT) with the level terms (LLT) of the training set resulting in a finetuned model;
d. saving the finetuned model for mapping the textual description RT to a best-matching lowest level term LLT for later use;
e. reiterate training for next model for mapping the textual description RT to a best-matching lowest level term LLT and / or next pretrained transformer model transformer model.

In an embodiment, only training data relating to top-M candidate classes from a single finetuned transformer model or deep ensemble model are used for training of the xTARS model.

In an embodiment, a xTARS model is trained to produce a probability of classification for each of the top-M candidates.By way of changing the training data and/or the random seed initialization, one or more trained xTARS model algorithm can be trained for mapping a textual description (RT) to lowest level terms (LLT).

In an embodiment, in step c), each trained xTARS model for mapping the textual description RT to lowest level terms LLT is validated, by way of:

- Acquiring a validation set medical event records, the validation set comprising randomly selected records wherein textual description of a medical event (RT) and corresponding lowest level terms (LLT) have been defined by a human coder;
- Predicting the lowest level term (LLT) for the textual description of a medical event (RT) using the trained xTARS model for mapping a textual description RT to lowest level terms LLT;
- Comparing the predicted LLTs with acquired LLTs of the validation set;
- Calculating an accuracy value for the trained xTARS model at stake.

In an embodiment, the trained xTARS models with highest accuracy value are selected for each random seed and saved for later use.

In an embodiment, obtained multiple trained xTARS models can be combined following the deep ensemble procedure mentioned above to obtain best possible prediction xTARS-based algorithm.

As mentioned above, the one or more finetuned algorithms trained for mapping textual descriptions (RT) to a lowest level term (LLT) obtained and validated by the methods mentioned above, can be used in a computer implemented method for attribution of a standardized hierarchical alphanumerical term and code to a textual description of a medical event (RT).

In an embodiment, the one or more finetuned algorithms for mapping textual descriptions (RT) to the lowest level terms (LLT) are one or more trained deep ensembles or one or more trained xTARS model algorithms.

In an embodiment, one finetuned transformer model, one or more trained deep ensembles is used for selection of a subset of best lowest level term (LLT) proposals, and one or more xTARS model is used to for mapping textual descriptions (RT) to one lowest level term (LLT) out of said subset.

In an embodiment, wherein each finetuned algorithm for mapping textual descriptions (RT) to the lowest level terms (LLT) is providing a probability vector of classification comprising a probability value for each lowest level term (LLT) of the ontology.

In other words, the term "probability vector of classification" as used herein refers to a vector with non-negative entries that add up to one returned by a classification model with number of components equal to the number of possible classes (LLT). The i-th value of the probability vector of classification represents the probability that the i-th class is the correct class according to the model that returned the probability vector.

The term "highest probability value in the probability vector of classification" as used herein refers to the highest value among the components of the probability vector of classification.

In an embodimentA a consensus between the multiple algorithms is formed by way of one or more of the following steps:

a) the proposal for lowest level term (LLT) with highest probability value in the probability vector(s) of classification can be selected;

b) several probability vectors can be averaged and proposal for lowest level term (LLT) with highest probability value can be selected in the averaged probability vector;

c) the vectors of probability can be averaged for all finetuned models based on the same pretrained transformer model (e.g. sciBERT, bioBERT or PubMedBERT), resulting in a subset of averaged probability vectors (one per pretrained transformer model) and proposal for lowest level term (LLT) with highest probability value can selected from the subset of averaged probability vectors.

In an embodiment an uncertainty is evaluated for the algorithm which led to the selected lowest level term with highest probability value. The uncertainty of each model for mapping the textual description (RT) with the lowest level terms (LLT) can be predictive entropy or the highest probability value.

Since a deep ensemble model is not specialized to learn from few training samples, its accuracy can be lower for rare classes. In addition, the deep ensemble model is also generally more uncertain on samples from rare classes.

In an embodiment, the predictions from the deep ensemble(s) can be enhanced using xTARS model(s).

In such an embodiment, an uncertainty threshold can be defined; for proposals given by models with uncertainty above this threshold, the deep ensemble prediction can be overwritten by an xTARS prediction. This threshold can also be set to zero; in that case, the prediction for xTARS is kept for all samples, irrespective of their uncertainty.

In an embodiment, the method can be configured to be always causing display of the selected one or more proposals for lowest level term (LLT) to a user for approval or rejection.

In another embodiment, causing display of the selected one or more proposals for lowest level term (LLT) to a user for approval or rejection may be triggered only if the uncertainty of the model(s) providing the selected one more proposal is above a predefined uncertainty threshold. In cases wherein the method can select one lowest level term with acceptable uncertainty, the machine-selected proposal for lowest level term (LLT) to the record relating to the textual description of a medical event (RT).As already mentioned, the present invention is preferably used for attribution of a standardized hierarchical alphanumerical terms and codes to a textual description of a medical event (RT), wherein said alphanumerical terms and codes are organized in lowest level terms (LLT). Medical coding in the MedRA ontology was used as an example. This preferred embodiment is explained in somewhat greater detail below, but without any intention of restricting the invention to this embodiment.

The method mentioned above may be used for medical coding of a medical event in another ontology or in several ontologies. It may also be used to translate medical coding from an ontology into another ontology.

Further potential use may be for attribution of medical coding from electronic health record narratives, in other words for transformation of electronic health records into a standardize form.

Further objects of the present invention are:

- A data processing system comprising a processor adapted to/configured to perform the method for attribution of a standardized hierarchical alphanumerical terms and codes to a textual description of a medical event (RT), wherein said alphanumerical terms and codes are organized in lowest level terms (LLT) of an ontology described above.
- A computer program [product] comprising instructions which, when the program is executed by a computer, cause the computer to carry out the method for attribution of a standardized hierarchical alphanumerical terms and codes to a textual description of a medical event (RT), wherein said alphanumerical terms and codes are organized in lowest level terms (LLT) of an ontology described above.
- A computer-readable [storage] medium comprising instructions recorded thereon which, when executed by a computer, cause the computer to carry out [the steps of] the method for attribution of a standardized hierarchical alphanumerical terms and codes to a textual description of a medical event (RT), wherein said alphanumerical terms and codes are organized in lowest level terms (LLT) of an ontology described above.


Further

- A data processing system comprising a processor adapted to/configured to perform the method for training of an algorithm for mapping a textual description of a medical event (RT) to lowest level terms (LLT) of an ontology described above.
- A computer program [product] comprising instructions which, when the program is executed by a computer, cause the computer to carry out the method for training of an algorithm for mapping a textual description of a medical event (RT) to lowest level terms (LLT) of an ontology described above.
- A computer-readable [storage] medium comprising instructions recorded thereon which, when executed by a computer, cause the computer to carry out [the steps of] the method for training of an algorithm for mapping a textual description of a medical event (RT) to lowest level terms (LLT) of an ontology described above.


The use of terms "a" and "an" and "the" and similar referents in the context of describing the invention (especially in the context of the following claims) are to be construed to cover both the singular and the plural, unless otherwise indicated herein or clearly contradicted by context. The terms "comprising", "including", "having", and "containing" are to be construed as open-ended terms (i.e. meaning "including but not limited to") unless otherwise noted.

The use of any and all examples, or exemplary language (e.g., "such as") provided herein, is intended merely to better illuminate the invention and does not pose a limitation on the scope of the invention unless otherwise claimed. No language in the specifications should be constructed as indicating any non-claimed element as essential to the practice of the invention.

Preferred embodiments of this invention are described, including the best mode known to the inventors for carrying out the invention.

Variations of those preferred embodiments can become apparent to those of ordinary skilled artisans to employ such variations as appropriate, and the inventors intend for the inventions to be practiced otherwise than specifically described herein.

Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

If steps are stated in an order in the present description or in the claims, this does not necessarily mean that the invention is restricted to the stated order. On the contrary, it is conceivable that the steps are also executed in a different order or else in parallel to one another, unless one step builds upon another step, this absolutely requiring that the building step be executed subsequently (this being, however, clear in the individual case). The stated orders are thus preferred embodiments of the invention.

DETAILLED DESCRIPTION OF THE INVENTION

Figure 1 shows a summary diagram for the creation of the training, validation, and test set from raw data via data processing and augmentation.

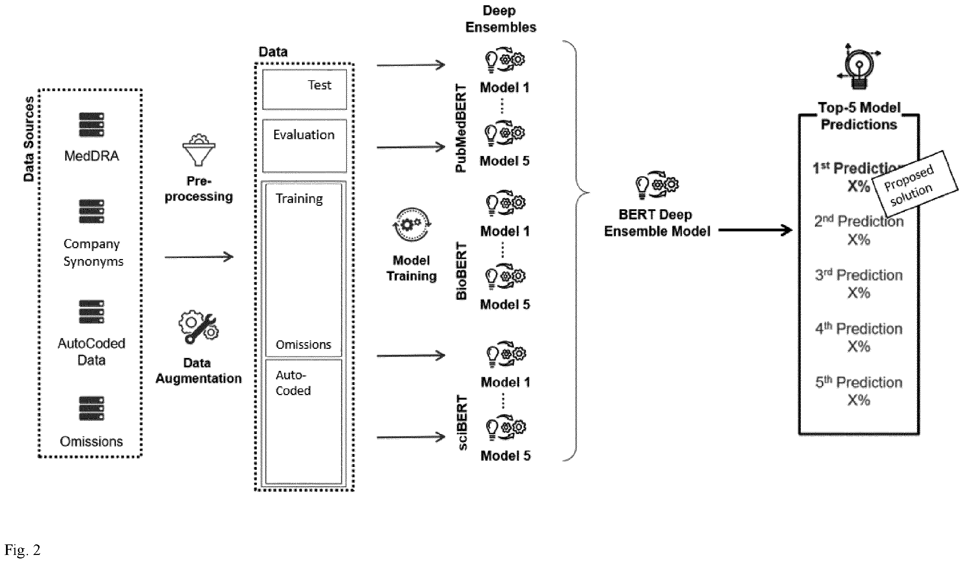

Figure 2 shows a summary diagram of the present invention from a raw training dataset to model prediction for the deep ensemble model.

Figure 3 shows a summary diagram of the present invention from a raw training dataset to model prediction for the xTARS model.

Fig. 4 shows a diagram of an embodiment of the method for attribution of a lowest level term (LLT) to a textual description of a medical event (RT).

Fig. 5 shows a diagram of an embodiment of the method for training algorithms for mapping a textual description of a medical event (RT) to a lowest level term (LLT)

Preferred embodiment of the algorithm used is depicted in Fig. 3.

A training dataset comprise the following data was used:

- Coded data;.
- Autocoded data;
- MedDRAOntology, wherein each LLT can be used also as RT in the training dataset; and
- Company synonyms: pairs of medical text descriptions and corresponding LLT.

To avoid redundancies in the data set, the training data set was low-cased, and only unique RTs were kept; if multiple RT/LLT pairs were present, the most recent pair were kept.

Then, the whole data set of RT/LLT pairs was split into training, validation, and test dataset. The most recent 5% of coded data were used as test dataset.

From the remaining coded data, a randomly sampled 10% of the coded data (excluding no data augmentation) was used as validation data.

The training data comprised all autocoded data, all RT originating from the ontology, the company synonyms, and the remaining coded data (~ 85%), plus augmented data.

Finally, the augmented samples were removed from the training data if their original sample was included in the validation data. This was done to avoid target leakage due to data augmentation, and enforce the independence of the validation data

Table 1: A sample of the data

| Reported term (RT) | Lowest level term (LLT) name | Preferred term (PT) name |
|---|---|---|
| on and off lethargy | lethargy | lethargy |
| scattered indeterminate subcentimeter pulmonary nodules | lung nodule | pulmonary mass |
| | chronic kidney disease | chronic kidney disease |
| ckd-unknown etiology | | |

(continued)

| Reported term (RT) | Lowest level term (LLT) name | Preferred term (PT) name |
| --- | --- | --- |
| elective left fem-pop bypass graft | femoropopliteal artery bypass | peripheral artery bypass |
| osteomyelitis of the left metatarsal | osteomyelitis of the foot | osteomyelitis |
| right foot second toe gangrene | gangrene toe | gangrene |
| worsenin renal function | renal function aggravated | renal impairment |
| oliguric acute kidney injury/ckd | acute oliguric renal failure | acute kidney injury |
| urinary tract infection [enterobacter cloacae] | urinary tract infection bacterial | urinary tract infection bacterial |
| skin defect [no split] | skin disorder | skin disorder |
| haemangioma th12 | spinal haemangioma | haemangioma of bone |
| pain of right thigh to the knee | unilateral leg pain | pain in extremity |

Three pretrained transformer models were used: bioBERT, PubMedBERT and sciBERT.

For each pretrained transformer model, five different models which differ only in the random seed initialization were trained, and then an average within models of the same type was performed. As a result, three different deep ensembles, one for each language model were obtained.

Training was performed on the training dataset for 20 epochs with Adam optimizer with a learning rate of 1e-4 and batch size of 512.

Learning rate of 1e-5 and 5e-5 were also evaluated but yield a lower performance. The same hyper-parameters were used for each pretrained transformer model. Training a single model took approximately 7 hours on 4 Tesla V100 GPUs. We selected the model with the highest accuracy on the validation (holdout) data were selected. The model was also evaluated on the test data to test the model's performance stability.

To obtain a principled uncertainty estimates, the concept of predictive entropy was used. Predictive entropy has its mathematical foundation from information theory (C.E. Shannon, 1948, A mathematical theory of communication. The Bell System Technical Journal, 27(3):379-423 ) and captures the average amount of information contained in the predictive distribution. For a given test point x, the predictive entropy reads (Y. Gal, 2016, Uncertainty in deep learning, PhD Thesis, https://mlg.eng.cam.ac.uk/yarin/thesis/thesis.pdf):

$$\mathbb{H}\left[y|x, \mathcal{D}_{\text{train}}\right] =$$
$$= -\sum_{c} p\left(y = c|x, \mathcal{D}_{\text{train}}\right) \log p\left(y = c|x, \mathcal{D}_{\text{train}}\right)|$$

$$(1)$$

where the sum is performed over all possible classes c of the output y, and Dtrain is the training dataset. In our case, p is the classification probability of the deep ensemble model, obtained by averaging the probability vector of classification of the models belonging to the ensemble.

The predictive entropy quantifies the prediction confidence: the larger (smaller) the predictive entropy, the more uncertain (certain) the prediction is. The maximum of the predictive entropy is attained when all probabilities p are equal, while it is zero when one probability is equal to one and all the rest are zero.

To obtain one prediction deep ensembles from different pretrained transformer models were combined.

To this end, two strategies were adopted: average of probability vector of classification, or prediction from the most certain model.

In the first approach, for each data point, the probability vectors of classification returned by the three deep ensembles were averaged. The predicted class (i.e., LLT name) was the class which had the highest prediction probability after the average is performed.

In the second approach, for each prediction, the uncertainty of each of the three ensembles are evaluated, and the class (i.e., LLT name) predicted by the most certain ensemble was returned. The most certain model can be selected either via predictive entropy (model with the lowest predictive entropy), or classification probability (model with the highest prediction probability).

Any of these strategies was found to be usable alone or and combined, depending on model performance and easiness of model deployment in a production environment.

xTARS models were trained a similar fashion as the deep ensemble model with the exception that only coded and autocoded data were used as training data; no data augmentation was used to reduce computational cost.

Multiple xTARS models were combined using the deep ensemble procedure presented above.

The predictions from the deep ensemble and the xTARS model were combined to achieve final best-matching proposal for LLT to a new medical event RT.

Since the deep ensemble model is not specialized to learn from few training samples, its accuracy is lower for rare classes. In addition, the model is also generally more uncertain on samples from rare classes. For these reasons, a predictive entropy threshold was used to extract uncertain samples: for sample above this threshold, the deep ensemble prediction was overwritten by xTARS.

In other words, first, BERT pretrained transformers or BERT-ensemble were used to propose a number K of best candidates LLT (K=5 was used), and these K best candidates were shown to a trained xTARS model. xTARS produced a probability of classification for each of the K candidates. The selected proposal for display was the class which has the highest probability according to xTARS.

In an example, this threshold was set to zero: the prediction for xTARS were kept for all samples, irrespective of their uncertainty.

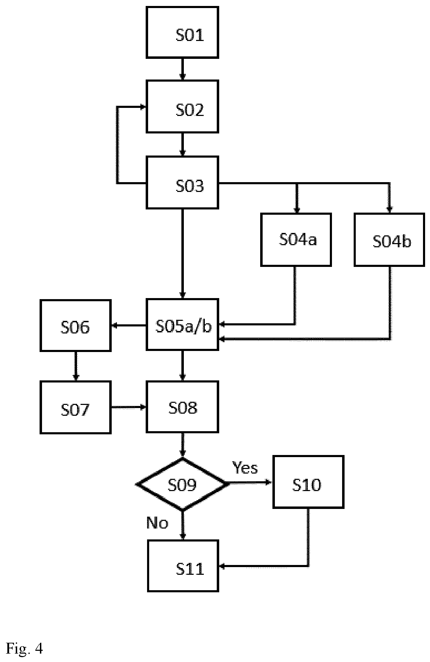

Fig. 4 shows a diagram of the method for attribution of a lowest level term (LLT) to a textual description of a medical event (RT), comprising the following steps:

S01 Acquiring a textual description of a medical event (RT) to be classified.

S02 Inputting RT into an algorithm finetuned for mapping textual descriptions (RT) to a lowest level term (LLT), said algorithm, comprising a pretrained transformer model finetuned for mapping textual descriptions (RT) to a lowest level term (LLT) and creating a probability vector of classification;

S03 Repeating S02 with further finetuned algorithms, so a list of probability vectors of classification corresponding to LLT proposals is obtained.

S04a Averaging the probability vectors; or

S04b Averaging the probability vectors for all finetuned algorithms based on the same pretrained transformer, so n average probability vectors of classification are obtained, wherein n is the number of pretrained transformers used.

S05a Selecting one proposal for lowest level term (LLT) with highest probability value in the probability vector of classification from S03 (single best value from all probability vectors), S04a (one best value from one averaged probability vector), S04b (single best value from n probability vectors).

OR

S05b Selecting a predefined number of k proposals for lowest level term (LLT) with highest probability values in the probability vector of classification from S03, S04a or S04b.

S06 Inputting k proposals of S05b into a trained xTARS model, producing a probability of classification for each of the k proposals.

S07 Selecting one proposal for lowest level term (LLT) with highest probability of classification for each of the k proposals.

S08 Evaluating uncertainty of the model(s) corresponding to selected proposal(s) for lowest level terms LLT from S05a or S07.

S09 Comparing the uncertainty of S08 and select the LLT(s) provided by the model(s) with the lowest uncertainty.

S10 Causing display of the selected one or more proposals for lowest level term (LLT) to a user if the uncertainty of the corresponding models is above a predefined threshold or if more than one proposal is selected in step S09.

S11 Saving the proposal for lowest level term (LLT) to a record relating to the textual description of a medical event (RT).

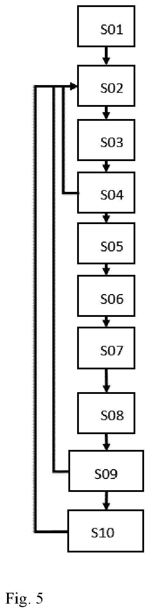

Fig. 5 shows a diagram of the method for training algorithms for mapping a textual description of a medical event (RT) to a lowest level term (LLT), comprising the following steps:

S01 Acquiring a training dataset of medical event records comprising textual description of a medical event (RT) and corresponding lowest level terms (LLT).

S02 Feeding the training set of medical records to a pretrained transformer model for differential weighting of the significance of each part of the textual descriptions of a medical event (RT).

S03 Initiating training of the model using a random seed.

S04 Reiterative training of further models with same random seed for multiple (best 20) epochs with Adam optimizer to obtain a set of finetuned models for mapping the textual description (RT) with the level terms (LLT).

S05 Acquiring a validation set medical event records, the validation set comprising records wherein a random subset of textual description of a medical event (RT) and corresponding lowest level terms (LLT) have been defined by a human coder.

S06 Predicting the lowest level term (LLT) for the textual description of a medical event (RT) using each finetuned model of S04 and calculating an accuracy value for the finetuned model at stake for the validation set.

S07 Selecting the finetuned model of S06 with the highest accuracy value.

S08 Saving the selected finetuned model and related pretrained transformer model for later use.

S09 Reiterating training for next model using a new random seed until a predefined number of random seeds is achieved.

S10 Reiterating S02 to S09 for next model(s) using next pretrained transformer model.

At the end of the training phase, a collection of finetuned models for mapping a textual description (RT) to lowest level terms (LLT) is obtained. Said collection can be used for prediction as described in Fig. 4.

A preferred embodiment of the present invention has been described in detail hereinabove with reference to the accompanying drawings, but the present invention is not limited to said example. It is obvious that persons having an ordinary level of knowledge in the technical field of the present invention could conceive of various variations or modifications within the scope of the technical concepts recited in the scope of the patent claims, and it is to be understood that these are also included within the technical scope of the present invention.

**Claims**

1.  Computer-implemented method for attribution of a standardized hierarchical alphanumerical term and code from an ontology of standardized terms and codes organized in lowest level terms (LLT) to a textual description of a medical event (RT), comprising the steps of:

    a) Acquiring a textual description of a medical event (RT);
    b) Selecting one or more proposals for lowest level term (LLT) for the textual description of a medical event (RT) using one or more algorithm based on one or more pretrained transformer model(s) finetuned for mapping textual descriptions (RT) to the lowest level terms (LLT);
    c) Optionally selecting one best matching proposal for lowest level term (LLT) by way of causing display of the selected one or more proposals for lowest level term (LLT) to a user for approval or rejection or automatically according to predefined criteria;
    d) Saving the best matching proposal for lowest level term (LLT) to a record relating to the textual description of a medical event (RT).

2.  The method according to claim 1, wherein the one or more pretrained transformer models are selected from a list comprising bioBERT, PubMedBERT and sciBERT.

3. The method according to one of the preceding claims, wherein the one or more finetuned algorithms for mapping textual descriptions (RT) to the lowest level terms (LLT) are one or more trained deep ensembles or one or more trained xTARS model algorithms.

4. The method according to one of the preceding claims, wherein one or more trained deep ensembles is used for selection of a subset of best lowest level term (LLT) proposals, and one or more xTARS model is used to for mapping textual descriptions (RT) to one lowest level term (LLT) out of said subset.

5. The method according to one of the preceding claims, wherein each finetuned algorithm for mapping textual descriptions (RT) to the lowest level terms (LLT) is providing a probability vector of classification comprising a probability value for each lowest level term (LLT) of the ontology.

6. The method according to claim 5, wherein the proposal for lowest level term (LLT) with highest probability value in the probability vector(s) of classification is selected.

7. The method according to claim 5, wherein several probability vectors are averaged and proposal for lowest level term (LLT) with highest probability value is selected in the averaged probability vector.

8. The method according to claim 5, wherein the vectors of probability are averaged for all finetuned models based on the same pretrained transformer model, resulting in a subset of averaged probability vectors and proposal for lowest level term (LLT) with highest probability value is selected from the subset of averaged probability vectors.

9. The method according to claim 5, wherein an uncertainty is evaluated for the algorithm which led to the selected lowest level term.

10. The method of claim 9, wherein the uncertainty of each model for mapping the textual description (RT) with the level terms (LLT) is predictive entropy or the highest probability value.

11. The method according to claim 5, wherein causing display of the selected one or more proposals for lowest level term (LLT) to a user for approval or rejection is conducted only if the uncertainty of the corresponding model is above a predefined threshold.

12. Computer-implemented method for training of an algorithm for mapping a textual description of a medical event (RT) to lowest level terms (LLT) of an ontology, comprising the steps of:

    a. acquiring a training dataset of medical event records, the training set comprising records showing:

        - pairs of textual description of a medical event (RT) and corresponding lowest level terms (LLT) that have been defined by a human coder;
        - pairs of textual description of a medical event (RT) and corresponding lowest level terms (LLT) that have been coded by a rule-based autocoding system;
        - the ontology as medical event (RT) and lowest level terms (LLT); and /or
        - randomized pairs of textual descriptions of a medical event (RT) comprising one random character change and / or one word split and corresponding lowest level terms (LLT);

    b. feeding the training set of medical records to a pretrained transformer model for differential weighting of the significance of each part of the textual descriptions of a medical event (RT);
    c. initiating training of the model using a random seed to obtain a finetuned model for mapping a textual description (RT) with the level terms (LLT);
    d. saving the finetuned model for mapping the textual description (RT) to a lowest level term LLT for later use;
    e. reiterating step c) to d) for training of next model for mapping the textual description RT to a lowest level term LLT.

13. The method of claim 12, wherein the training set further comprises pairs of company synonyms for textual description of a medical event (RT) and corresponding lowest level terms (LLT).

14. The method of claim 12, wherein step c) is reiterated for one or more random seeds resulting, for each random seed, in multiple finetuned models for mapping a textual description (RT) with lowest level terms (LLT).

**15.** The method of claim of 12, wherein the finetuned model for a mapping a textual description (RT) to lowest level terms (LLT) is validated, by way of:

- Acquiring a validation set of medical event records, the validation set comprising randomly selected records wherein textual description of a medical event (RT) and corresponding lowest level terms (LLT) have been defined by a human coder;
- Predicting for each record one best-matching lowest level term (LLT) for the textual description of a medical event (RT) using the finetuned model for mapping a textual descriptions (RT) to lowest level terms (LLT), wherein said finetuned model is providing a probability vector of classification comprising a probability value for each lowest level term (LLT) of the ontology and the lowest level term (LLT) with highest probability value in the probability vector is selected;
- For each record of the validation set, comparing the selected lowest level term (LLT) with acquired lowest level term (LLT) of the validation set;
- Calculating an accuracy value for the finetuned model at stake based on said comparison;
- For each random seed, selecting the finetuned model with highest accuracy value; and
- Saving the selected finetuned model for later use.

**16.** The method of claim 12, wherein for each pretrained transformer model, more than one random seed is used.

**17.** The method of one of the preceding claims, wherein multiple pretrained transformer models are used, and multiple finetuned models for mapping a textual description (RT) with the level terms (LLT) are generated for each pretrained transformer model.

**18.** A data processing system comprising a processor adapted to/configured to perform the method for attribution of a standardized hierarchical alphanumerical term and code from an ontology of standardized terms and codes organized in lowest level terms (LLT) to a textual description of a medical event (RT) according to one of the claims 1 to 11.

**19.** A computer program comprising instructions which, when the program is executed by a computer, cause the computer to carry out the method for attribution of a standardized hierarchical alphanumerical term and code from an ontology of standardized terms and codes organized in lowest level terms (LLT) to a textual description of a medical event (RT) according to one of the claims 1 to 11.

**20.** A computer-readable storage medium comprising instructions recorded thereon which, when executed by a computer, cause the computer to carry out the method for attribution of a standardized hierarchical alphanumerical term and code from an ontology of standardized terms and codes organized in lowest level terms (LLT) to a textual description of a medical event (RT) according to one of the claims 1 to 11.

**21.** A data processing system comprising a processor adapted to/configured to perform the method for training of an algorithm for mapping a textual description of a medical event (RT) to lowest level terms (LLT) of an ontology according to one of the claims 12 to 17.

**22.** A computer program comprising instructions which, when the program is executed by a computer, cause the computer to carry out the method for training of an algorithm for mapping a textual description of a medical event (RT) to lowest level terms (LLT) of an ontology according to one of the claims 12 to 17.

**23.** A computer-readable storage medium comprising instructions recorded thereon which, when executed by a computer, cause the computer to carry out the method for training of an algorithm for mapping a textual description of a medical event (RT) to lowest level terms (LLT) of an ontology according to one of the claims 12 to 17.

Fig. 1

Fig. 2

Fig.3

Fig. 4

Fig. 5

Europäisches
Patentamt

European
Patent Office

Office européen
des brevets

# EUROPEAN SEARCH REPORT

Application Number

EP 22 15 1155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOPEZ-GARCIA GUILLERMO ET AL: "Transformers for Clinical Coding in Spanish", IEEE ACCESS, IEEE, USA, vol. 9, 13 May 2021 (2021-05-13), pages 72387-72397, XP011855819, DOI: 10.1109/ACCESS.2021.3080085 [retrieved on 2021-05-19] * figure 3 * * sections II-IV * | 1-23 | INV. G06N3/08 G06N3/04 G16H70/60 ADD. G06N5/02 |
| A | JI SHAOXIONG ET AL: "Does the magic of BERT apply to medical code assignment? A quantitative study", COMPUTERS IN BIOLOGY AND MEDICINE, vol. 139, 1 December 2021 (2021-12-01), page 104998, XP055932773, US ISSN: 0010-4825, DOI: 10.1016/j.compbiomed.2021.104998 * figure 1 * | 2 | |

TECHNICAL FIELDS
SEARCHED (IPC)

G06N
G16H

The present search report has been drawn up for all claims

| Place of search | Date of completion of the search | Examiner |
|---|---|---|
| The Hague | 17 June 2022 | Theissing, Simon |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

### Non-patent literature cited in the description

- **M. E. PETERS et al.** Deep contextualized word representations. *NAACL-HLT,* 2018, 2227-2237
- **J. DEVLIN et al.** *Computational Linguistics,* 2018
- **A.VASWANI et al.** Attention is all you need. *Advances in Neural Information Processing Systems,* 2017, vol. 30, 5998-6008
- **Y. GU et al.** Domain-specific language model pre-training for biomedical natural language processing. *CoRR,* 2020
- **I. CHALKIDIS et al.** An empirical study on largescale multi-label text classification including few and zero-shot labels. *Proceedings of the 2020 Conference on Empirical Methods in Natural Language Processing (EMNLP),* 2020, 7503-7515
- **J. LEE et al.** BioBERT: a pre-trained biomedical language representation model for biomedical text mining. *Bioinformatics,* 2019, vol. 36 (4), 1234-1240
- **I. BELTAGY et al.** SciBERT: A pretrained language model for scientific text.In Proceedings of the 2019 Conference on Empirical Methods in Natural Language Processing and the 9th International Joint Conference on Natural Language Processing (EMNLP-IJCNLP). *Association for Computational Linguistics,* 2019, 3615-3620
- **W. AMMAR et al.** Construction of the literature graph in semantic scholar. In Proceedings of the 2018 Conference of the North American Chapter of the Association for Computational Linguistics: Human Language Technologies. *Association for Computational Linguistics,* 2018, vol. 3, 84-91
- **B. LAKSHMINARAYANAN et al.** Simple and scalable predictive uncertainty estimation using deep ensembles. In Proceedings of the 31st International Conference on Neural Information Processing Systems. *NIPS,* 2017, vol. 17, 6405-6416
- **S. FORT et al.** Deep ensembles: A loss landscape perspective. *arXiv,* 2020
- **Y. GAL et al.** Dropout as a bayesian approximation: Representing model uncertainty in deep learning. *Proceedings of the 33rd International Conference on International Conference on Machine Learning,* 2016, vol. 48, 1050-1059
- **K. HALDER.** Task-aware representation of sentences for generic text classification. In Proceedings of the 28th International Conference on Computational Linguistics. *International Committee on Computational Linguistics,* 2020, 3202-3213
- **C.E. SHANNON.** A mathematical theory of communication. *The Bell System Technical Journal,* 1948, vol. 27 (3), 379-423
- **Y. GAL.** Uncertainty in deep learning. *PhD Thesis,* 2016, https://mlg.eng.cam.ac.uk/yarin/thesis/thesis.pdf